# EUROPÄISCHE PATENTSCHRIFT

(11) **EP 3 481 502 B1**
(45) Veröffentlichungstag und Bekanntmachung des Hinweises auf die Patenterteilung: **18.03.2020**
(21) Anmeldenummer: 17735546.8
(22) Anmeldetag: 06.07.2017
(51) Int. Cl.: A61N 1/375, H01R 13/52, H01R 13/02

(54) **IMPLANTIERBARER, ELEKTROMECHANISCHER STECKVERBINDER**
IMPLANTABLE ELECTROMECHANICAL PLUG CONNECTOR
CONNECTEUR ÉLECTROMÉCANIQUE ENFICHABLE IMPLANTABLE

(30) Priorität: 06.07.2016 DE 102016212332
(43) Veröffentlichungstag der Anmeldung: 15.05.2019
(73) Patentinhaber: Neuroloop GmbH, 79108 Freiburg (DE)
(72) Erfinder: KIMMIG, Fabian, 79110 Freiburg (DE); BORETIUS, Tim, 79098 Freiburg (DE)
(74) Vertreter: Rösler, Uwe
(86) Internationale Anmeldenummer: PCT/EP2017/066925
(87) Internationale Veröffentlichungsnummer: WO 2018/007517

(56) Entgegenhaltungen:
- WO-A1-2008/025159
- WO-A1-2009/045772
- WO-A2-2009/126872
- US-A1- 2008 246 231

## Beschreibung

### Technisches Gebiet

Die Erfindung bezieht sich auf einen implantierbaren, elektromechanischen Steckverbinder mit einem Stecker- und einem Buchsenteil.

Elektromechanische Steckverbinder betreffen im Allgemeinen zwei mechanisch lösbarfest ineinander fügbare Komponenten zu Zwecken einer elektrischen Energie- und/oder Signalübertragung und unterliegen in Abhängigkeit ihrer Ausbildung und Anwendung spezifischen betriebssicherheitsrelevanten Anforderungen. Handelt es sich um implantierbare Steckverbinder, so müssen diese den Anforderungen für aktive, implantierbare Medizinprodukte genügen, die einem dauerhaft feuchten Milieu ausgesetzt sind und diesem für eine möglichst lange Zeitdauer unbeschadet gegenüber Feuchtigkeits- bzw. Wassereintritt in das Innere des jeweiligen Implantates standhalten sollen.

Besonders kritisch sind bei an sich bekannten implantierbaren Steckverbindern die Fügeabschnitte längs der sich die Steckverbinderkomponenten zumeist form- und kraftschlüssig an- und ineinanderfügen. Die besondere Herausforderung bei der Konstruktion und Ausgestaltung derartiger Steckverbinder besteht darin, ein Eindringen von Wasser bzw. Feuchtigkeit in und durch die innerhalb eines Steckverbinders enthaltenen Grenzflächen zwischen Stecker- und Buchsenteil möglichst langfristig zu unterbinden, um Wasser- bzw. Feuchtigkeitskontakt mit den innerhalb eines Steckverbinders enthaltenen elektrischen Strukturen zu vermeiden. So führen Wasserkontakte an elektrisch leitenden, zumeist aus metallischem Material bestehenden Leitungs- und Elektrodenstrukturen zu irreversiblen Degradationserscheinungen und einer damit verbundenen Beeinträchtigung der elektrischen Energie- und Signalübertragungseigenschaften. Darüber hinaus vermag die Gegenwart von Wasser bzw. Feuchtigkeit Ablösungen zwischen den innerhalb des Steckverbinders enthaltenen, metallischen Strukturen und den diese unmittelbar umgebenden, zumeist aus polymeren Materialien bestehenden Oberflächen der Steckverbinderkomponenten hervorzurufen und somit die Lebensdauer derartiger Steckverbinder herabzusetzen.

### Stand der Technik

Eine bekannte elektrische Durchführung zum Einsatz in einem Gehäuse einer aktiven implantierbaren medizinischen Vorrichtung ist in der Druckschrift DE 10 2011 009 857 B4 beschrieben, deren wenigstens eine durch die Gehäusewand hindurchreichende elektrische Verbindungsstruktur hermetisch von einem Grundkörper umgeben ist, der sich fluiddicht an die Gehäusewand anschmiegt. Zudem mündet die elektrische Verbindungsstruktur an einen elektrischen Anschlusskontakt innerhalb eines als Steckerbuchse ausgebildeten und fluiddicht an die Gehäusewand angefügten Kopfteils. Die über wenigstens eine elektrische Anschlussstruktur verfügende Steckerbuchse ist als Teil einer an sich bekannten Standardsteckverbindung ausgebildet.

Der Druckschrift EP 0 910 435 B1 ist eine elektrische Anschluss-Buchse für einen implantierbaren Herzschrittmacher zu entnehmen, die über eine elastisch verformbare Einsteckhülse verfügt, die im entspannten Zustand eine gekrümmte Form aufweist. Nach Einstecken eines geradzylinderförmig ausgebildeten Kontaktstiftes in die Hülse wird diese zwanghaft verformt und kommt unter Ausbildung einer weitgehend fluiddichten Verbindung an Außenbereiche des geradförmig ausgebildeten Kontaktstiftes zur Anlage.

Der Druckschrift DE 10 2012 020 260 B1 ist eine implantierbare subkutane elektrische Steckdose sowie ein diesbezüglicher perkutaner Stecker zu entnehmen, der eine, vorzugsweise mehrere perkutan in entsprechende trichterförmige Ausnehmungen innerhalb der Steckerbuchse zur Kontaktierung vorgesehener elektrischer Anschlussstrukturen vorsieht. Die implantierbare, subkutane, elektrische Steckdose weist ein Steckdosengehäuse auf, durch das elektrische Zu- und Ableitungen zur Energie- und Signalversorgung an wenigstens ein implantiertes medizinisches Gerät geführt wird.

Die Druckschrift DE 20 2007 019 606 U1 beschreibt eine Kontaktbuchse zum Anschluss einer Elektrodenleitung an ein implantierbares medizinisches Gerät mit einem Buchsengehäuse, das eine Steckeraufnahme mit einem längsgestreckten Aufnahmeraum aufweist. Die Steckeraufnahme besteht aus einem aus einer dauerelastischen Masse gebildeten Gussteil, in das elektrische Kontaktelemente aus elektrisch leitendem Material eingesetzt sind.

Der Druckschrift EP 0 811 397 B1 ist eine implantierbare Einheit mit mindestens einer Kontaktanordnung zur Verbindung einer in einem Gehäuse hermetisch dicht untergebrachten elektrischen Vorrichtung mit mindestens einem aus dem Gehäuse heraus geführten Anschlusskabel beschrieben, das von einem aus unelastischem Material gefertigten Formkörper umgeben ist und eine freizugängliche Elektrodenfläche besitzt, an die presskraftbeaufschlagt ein Gegenkontakt zu einem weiterführenden Anschlusskabel in Anlage gebracht wird, der von einem elastischen Material umgeben ist, in das eine den Kontaktbereich peripher umgebende rippenartige Erhöhung seitens des Formkörpers unter Ausbildung einer Formschlussverbindung eindringt.

Die Druckschrift JP 2013-094 456 A offenbart einen implantierbaren Steckverbinder mit einem Stecker- und Buchsenteil, bei dem die Anzahl der Stecker-seitigen elektrischen Kontaktstifte längs eines Querschnittsbereiches der Steckereinheit angebracht ist. Das Zusammenfügen der Kontaktstifte mit den Buchsen-seitig angebrachten Kontakthülsen erfolgt durch axiales Ineinanderfügen. Somit ist die Anzahl an elektrischen Kontakten aufgrund eines möglichst kleinen Durchmessers des Steckverbinders signifikant limitiert.

Die Druckschrift US 2005/0118887 A1 beschreibt einen implantierbaren Steckverbinder mit einer Vielzahl von Elektrodenkontakten. Die schubladenartig ausgebildete Steckereinheit weist an einer Oberseite Elektrodenkontakte auf, die im eingefügten Zustand innerhalb des Buchsenteils mittels eines orthogonal auf die Oberseite wirkenden Klemmmechanismusses gegen die die Buchsenteil-seitig angeordneten Gegenelektrodenflächen gepresst werden. Dies erfordert beim inkorporalen Applizieren des Steckverbinders einen zusätzlichen Arbeits- und Instrumentenaufwandes sowie einen hierfür nötigen Zugang und Raum zum Betätigen des bspw. als Inbusschraube ausgebildeten Klemmmechanismus.

Die Druckschrift WO 2008/025159 A1 offenbart einen Steckverbinder mit einem Steckerteil, dessen Fügeabschnitt stirnseitig axial in eine Ausnehmung eines Buchsenteils einfügbar ist. Die hierbei jeweils paarweise in gegenseitigen Kontakt kommenden Elektroden- und Gegenelektrodenkörper kontaktieren sich durch axiales Ineinandergreifen.

In ähnlicher Weise ist eine in der Druckschrift WO 2009/045772 A1 beschriebene Steckverbindereinheit aufgebaut, die ein aufklappbares Steckergehäuse aufweist, in dem Gegenelektrodenkontakte enthalten sind, in das ein Steckerteil einlegbar ist, dessen Elektrodenkörper beim Einlegen in das aufgeklappte Steckergehäuse dort befindliche Gegenelektroden kontaktieren.

### Darstellung der Erfindung

Der Erfindung liegt die Aufgabe zugrunde, einen implantierbaren, elektromechanischen Steckverbinder mit einem Stecker- und einem Buchsenteil derart auszubilden, so dass im implantierten Zustand mit ineinandergefügtem Stecker- und Buchsenteil der Steckverbinder über ein hohes Maß an Dichtheit verfügen soll, sodass feuchtigkeitsbedingte Materialdegradationen von an innerhalb des Steckverbinders vorhandenen elektrisch leitenden Strukturen vermieden werden sollen. Der implantierbare Steckverbinder soll darüber hinaus leicht und ohne zusätzliche Fügekomponenten intrakorporal manuell geschlossen bzw. geöffnet werden können. Ferner sollen Fehlbedienungen sowie auch bedienungsbedingte Beschädigungen an Steckverbinder-internen Elektrodenstrukturen ausgeschlossen werden. Schließlich soll es möglich sein trotz einer kompakten und kleinbauenden Raumform des Steckverbinders eine Vielzahl von elektrischen Übertragungsleitungen mit dem Steckverbinder elektrisch zu kontaktieren.

Die Lösung der der Erfindung zugrunde liegenden Aufgabe ist im Anspruch 1 angegeben. Den erfindungsgemäßen Gedanken in vorteilhafter Weise weiterbildende Merkmale sind Gegenstand der Unteransprüche sowie der weiteren Beschreibung insbesondere unter Bezugnahme auf die illustrierten Ausführungsbeispiele zu entnehmen.

Der implantierbare Steckverbinder weist einen Stecker- sowie einen Buchsenteil auf, von denen das Steckerteil wenigstens einen Fügeabschnitt besitzt, der vollständig in eine einseitig offen ausgebildete Einschuböffnung innerhalb des Buchsenteils einführbar ist und über wenigstens eine elektrisch isolierende Oberfläche verfügt, die wenigstens einen Elektrodenkörper mit einer frei zugänglichen Elektrodenoberfläche aufweist. Das Buchsenteil weist innerhalb der einseitig offen ausgebildeten Einschuböffnung wenigstens einen die Einschuböffnung zumindest bereichsweise seitlich begrenzenden elektrisch isolierenden Wandabschnitt auf, dessen Oberfläche wenigstens einen Gegenelektrodenkörper mit einer frei zugänglichen Gegenelektrodenoberfläche vorsieht, wobei der Buchsenteil-seitige, elektrisch isolierende seitliche Wandabschnitt der Steckerteil-seitigen, elektrisch isolierenden Oberfläche des Fügeabschnittes im ineinandergefügten Zustand derart zugewandt orientiert ist, so dass sich die Gegenelektroden- und die Elektrodenoberfläche unter Ausbildung eines elektrischen Flächenkontaktes berühren. Zudem ist der wenigstens eine Steckerteil-seitige Elektrodenkörper erhaben gegenüber der elektrisch isolierenden Oberfläche des Fügeabschnittes ausgebildet und/oder der wenigstens eine Buchsenteil-seitige Gegenelektrodenkörper erhaben gegenüber der Oberfläche des elektrisch isolierenden Wandabschnittes ausgebildet. Ferner ist zumindest bereichsweise zwischen der Steckerteil-seitigen, elektrisch isolierenden Oberfläche des Fügeabschnittes sowie der Buchsenteil-seitigen Oberfläche des elektrisch isolierenden Wandabschnittes wenigstens eine elektrisch isolierende Polymerschicht angeordnet, die die sich berührenden Gegenelektrodenoberfläche sowie Elektrodenoberfläche seitlich vollständig umfasst. Lösungsgemäß ist der Steckerteil im Rahmen eines Multilayer-Prozesses hergestellt ist und sieht eine Vielzahl von elektrisch leitenden Schichten, die jeweils elektrisch voneinander isoliert, in einem Stapelschichtverbund angeordnet sind, vor. Die einzelnen elektrisch leitenden Schichten münden jeweils elektrisch isoliert voneinander an der wenigstens einen elektrisch isolierenden Oberfläche jeweils unter Ausbildung eines Kontaktbereiches, wobei die Kontaktbereiche jeweils mit einem über die elektrisch isolierende Oberfläche erhabenen Elektrodenkörper kontaktiert sind.

Die wenigstens eine elektrisch isolierende Polymerschicht, besteht vorzugsweise aus einem elastischen Material, bspw. aus einem elektrisch isolierendem Elastomer, und ist fest entweder am elektrisch isolierenden Wandabschnitt oder an der elektrisch isolierenden Oberfläche des Fügeabschnittes gefügt.

Vorzugsweise besitzt die wenigstens eine elektrisch isolierende Polymerschicht eine Schichtdicke, die wenigstens einer orthogonalen Erhabenheit des wenigstens einen Gegenelektrodenkörpers gegenüber der Oberfläche des elektrisch isolierenden Wandabschnittes oder des wenigstens einen Elektrodenkörpers gegenüber der elektrisch isolierenden Oberfläche des Fügeabschnittes entspricht. Auf diese Weise ist sichergestellt, dass zum einen die sich kontaktierenden Elektroden- und Gegenelektrodenkörper im gefügten Steckverbinder-Zustand jeweils vollumfänglich von der Polymerschicht berührend umfasst sind, zum anderen schmiegt sich die Polymerschicht flächig an die Oberfläche des elektrisch isolierenden Wandabschnittes oder an die elektrisch isolierende Oberfläche des Fügeabschnittes fluiddicht an, je nachdem ob die wenigstens eine Polymerschicht Steckerteil- oder Buchsenteil-seitig fest angebracht ist.

Zur Verbesserung bzw. Erhöhung der Sperrwirkung gegen Eindringen von Feuchtigkeit in den Steckverbinder, sind die Größe und Form des Fügeabschnittes des Steckerteils, die Größe und Form der Einschuböffnung des Buchsenteils sowie die Größe und Form der elektrisch isolierenden Polymerschicht derart aufeinander abgestimmt dimensioniert, so dass im Zustand einer vollständigen Einführung des Steckerteil-seitigen Fügeabschnittes in die Einschuböffnung des Buchsenteils die wenigstens eine elektrisch isolierende Polymerschicht einer Kompressionskraft ausgesetzt ist, die zwischen der Oberfläche des elektrisch isolierenden Wandabschnittes und der elektrisch isolierenden Oberfläche des Fügeteils wirkt. Durch die Kompressionskraft wird die Polymerschicht mit einer erhöhten Anpresskraft an die jeweilige Oberfläche des Wandabschnittes bzw. der Oberfläche des Fügeabschnittes flächig angedrückt, wodurch sich eine fluiddichte Kraft- und Formschlussverbindung zwischen der Polymerschicht und der jeweiligen Oberfläche ausbildet.

Eine weitere bevorzugte Ausführungsform des Steckverbinders sieht vor, die Größe und Form sowohl des Fügeabschnittes, der Einschuböffnung, der wenigstens einen elektrisch isolierenden Polymerschicht und insbesondere der jeweiligen Elektroden- bzw. Gegenelektrodenkörper derart aufeinander abzustimmen, so dass die sich kontaktierenden Elektroden- und Gegenelektrodenoberflächen unter Anpresskraft gegenseitig zur Anlage gebracht werden. Wie die weiteren Ausführungen unter Bezugnahme auf ein konkretes Ausführungsbeispiel zeigen werden, kann die Anpresskraft vorzugsweise durch eine geeignet dimensionierte orthogonale Erhabenheit des wenigstens einen Elektrodenkörpers bzw. Gegenelektrodenkörpers gegenüber der jeweiligen Oberfläche in vorgebbarer Weise dimensioniert werden.

In einer weiteren bevorzugten Ausführungsform weist die Polymerschicht hygroskopisches Material auf, das bei Kontakt mit Feuchtigkeit bzw. Wasser einer Quellung und damit einer Volumenvergrößerung unterliegt, wodurch sich die Kompressionswirkung auf die Polymerschicht vergrößert und zugleich die Dichtwirkung verbessert wird.

Vorzugsweise eignet sich Polydimethylsiloxan, kurz PDMS, oder LCP (liquid crystal polymer) oder Parylene als Material für die wenigstens eine Polymerschicht. Selbstverständlich eignen sich auch alternative, biokompatible Polymer- bzw. Elastomermaterialien, bspw. Polyimid. Der vorstehend erläuterte Quelleffekt kann in vorteilhafter Weise durch Einlagerung von hygroskopischen Komponenten bzw. Verbindungen in der Polymerschicht realisiert werden, bspw. durch Einlagerung von Salzkristallen innerhalb einer PDMS-Schicht, oder durch Vorsehen von kristalloiden, hygroskopischen Zwischenschichten in einem schichtförmigen Polymer-Schichtenverbund. Als hygroskopische Komponenten eignen sich auch Silica-Gel, activated clay, chemical waterbinders, Zeolite, Zellulose.

Sämtliche Komponenten des lösungsgemäß ausgebildeten Steckverbinders sind aus biokompatiblen Materialien gefertigt, um so den medizinischen Zulassungsbedingungen gerecht zu werden. Als besonders geeignete Materialien für die Ausbildung des wenigstens einen Elektrodenkörpers sowie Gegenelektrodenkörpers eignen sich Metalle, wie bspw. Gold, Platin oder Iridium oder Metalllegierungen. Gleichfalls ist es möglich, anstelle von metallischen Elektrodenmaterialien auch leitfähige Polymermaterialien, wie bspw. Pedot zu verwenden.

In einer weiteren bevorzugten Ausführungsform ist der Steckerteil flächen- bzw. plattenförmig ausgebildet und verfügt über eine Ober- und Unterseite. Dem Fügeabschnitt des Steckerteils ist eine laterale Längs- und Quererstreckung zugeordnet, wobei die Länge des Fügeabschnittes in dessen Längserstreckung einer Einschubtiefe der Buchsenteil-seitig vorgesehenen Einschuböffnung entspricht, so dass im gefügten Zustand der Fügeabschnitt vollständig in die Einschuböffnung des Buchsenteils einmündet. Aus dem Buchsenteil ragt ein sich an den Fügeabschnitt einstückig anschließender Steckerabschnitt, an dem wenigstens ein elektrischer Kontakt vorgesehen ist, der elektrisch mit der wenigstens einen im Fügeabschnitt vorgesehenen Elektrodenoberfläche elektrisch verbunden ist. Der wenigstens eine elektrische Kontakt dient als elektrische Verbindungselektrode zu einem Leitungsdraht, der bspw. im Wege einer Lötverbindung mit dem elektrischen Kontakt verbunden ist.

Längs innerhalb der Buchsenteil-seitigen Einschuböffnung ist wenigstens eine wandseitige Einschubkulisse vorgesehen, längs der der Steckerteil-seitige Fügeabschnitt zwangsgeführt während des Einführens entlanggleitet. Die Einschubkulisse weist in einem bevorzugten Ausführungsbeispiel einen ersten Abschnitt auf, längs dem der Fügeabschnitt ausschließlich in seiner Längserstreckung einführbar ist. An den ersten Abschnitt längs der Einschubkulisse schließt sich ein zweiter, hinterer Abschnitt an, längs dem der Fügeabschnitt in dessen Längs- und Quererstreckung in die Einschuböffnung weiter einführbar ist. Durch den zweiten Abschnitt wird das Steckerteil relativ zum Buchsenteil diagonal zur Einstecklängsrichtung ausgelenkt und gelangt mit einem seitlichen Versatz relativ zur initialen Einstecklängsrichtung in eine Endposition.

Während des Einschubvorganges des Steckerteils in das Buchsenteil, bei dem der Steckerteil längs des ersten Abschnittes der Einschubkulisse zwangsgeführt wird, weisen der wenigstens eine Elektroden- und Gegenelektrodenkörper einen in Einschubrichtung seitlichen Abstand zueinander auf und gelangen nicht in gegenseitige Berührung. Insbesondere bei Vorsehen einer Vielzahl von Elektrodenkörpern auf dem Fügeabschnitt sowie einer entsprechenden Anzahl von Gegenelektrodenkörpern an wenigstens einer buchsenseitig vorgesehenen Oberfläche des elektrisch isolierenden Wandabschnittes gleiten die Elektrodenoberflächen der steckerseitig vorgesehenen Elektrodenkörper berührungslos an den Gegenelektrodenoberflächen der Buchsenteil-seitig vorgesehenen Gegenelektroden vorbei. Erst durch die den Fügevorgang des Steckerteils innerhalb der Einschuböffnung finalisierende Diagonal- bzw. Seitwärtsbewegung gelangen die Elektrodenoberflächen in Kontakt mit den diesen jeweils zugeordneten Gegenelektrodenoberflächen. Auf diese Weise werden unnötige verschleißbehaftete Übergleit- bzw. Schleifereignisse zwischen Elektroden- und Gegenelektrodenoberflächen während des Fügevorganges auf ein Minimum reduziert.

Hinzu kommt, dass durch die Seitwärtsbewegung der Steckerteil in die Endposition zumindest teilweise in einem sog. "Hinterschneidungsbereich" zu liegen kommt, in den durch geeignetes Design von Buchsenteil und Steckerteil ein Eindringen von Feuchtigkeit bzw. Wasser unmöglich oder zumindest erheblich erschwert wird. Insbesondere gelangen in der Endposition Bereiche der Seitenwände von Stecker- und Buchsenteil in einen innigen Flächenkontakt, wodurch die Eintrittsöffnung der Einschuböffnung vollständig fluiddicht zwischen Stecker- und Buchsenteil abgeschlossen wird.

Das Buchsenteil stellt im Wesentlichen eine Art Einsteckgehäuse für das Steckerteil dar. Das Gehäuse ist vorzugsweise aus einem starren formstabilen Material gefertigt, das aus einem biokompatiblen Material, vorzugsweise aus Titan oder einer Keramik besteht. Das Gehäuse umschließt die einseitig offen ausgebildete Einschuböffnung vollständig, sodass sich sämtliche auf das Gehäuse des Buchsenteils einwirkenden Kräfte längs in sich geschlossener Kraftpfade verteilen.

Vorzugsweise ist an dem die wenigstens eine frei zugängliche Gegenelektrodenoberfläche aufweisenden, elektrisch isolierenden Wandabschnitt innerhalb der Einschuböffnung des Buchsenteils die wenigstens eine Polymerschicht fest gefügt, die im Bereich der wenigstens einen Gegenelektrodenoberfläche eine Ausnehmung aufweist, so dass die wenigstens eine Gegenelektrodenoberfläche vollständig und nahtlos von der Polymerschicht umgeben ist.

Demgegenüber verfügt das Steckerteil wenigstens über einen über die elektrisch isolierende Oberfläche des Fügeabschnittes emporragenden Elektrodenkörper, der während des Einführens in die Einschuböffnung des Buchsenteils vor Erreichen der Endposition die elastische Polymerschicht lokal komprimiert und erst bei Erreichen der Endposition passgenau in die Ausnehmung innerhalb der Polymerschicht am Ort der Gegenelektrodenoberfläche einmündet. In diesem Zustand schmiegt sich die Polymerschicht nahtlos an die äußere Kontur des Elektrodenkörpers an.

Selbstverständlich sind alternative Ausführungsbeispiele denkbar, mit zwei sich innerhalb des Buchsenteils gegenüberliegend angeordneten, elektrisch isolierenden Wandabschnitten, an denen jeweils eine Polymerschicht angebracht ist. Ebenso ist es möglich eine Polymerschicht fest an der wenigstens einen elektrisch, isolierenden Oberfläche im Fügeabschnitt des Steckerteils anzubringen.

Der lösungsgemäße Steckverbinder zeichnet sich darüber hinaus durch eine kompakte und kleinbauende Raumform aus, die je nach Anzahl der innerhalb des Steckverbinders vorzusehenden Elektroden- und Gegenelektrodenkörpern eine Steckverbinderlänge und -breite von wenigen mm bis zu wenigen cm reichen kann. In Abhängigkeit von der Anzahl der im Fügeabschnitt des Steckerteils vorgesehenen Elektrodenkörper, zu denen entsprechend viele elektrische Kontakte im Steckerabschnitt vorzusehen sind, gilt es im Rahmen des Herstellungsprozesses elektrische Verbindungsstrukturen innerhalb des Steckerteils auszubilden. Die elektrischen Verbindungsstrukturen können als einzelne, elektrische Schichten innerhalb eines Stapelschichtenverbundes und/oder in Form von jeweils separat auf einer Zwischenschichtoberfläche aufgebrachte Leiterbahnen ausgebildet sein, zu deren elektrischen Kontaktierung zu den elektrischen Kontakten und den Elektrodenkörpern geeignete elektrische Verbindungen auszubilden sind.

In vorteilhafter Weise wird zur Herstellung des Steckerteils die Multilagen-Keramiktechnologie eingesetzt, vorzugsweise zur Herstellung sog. Niedertemperatur-Einbrand-Keramiken, LTCC, oder Hochtemperatur-Mehrlagenkeramiken, kurz HTCC. Für die Herstellung des wenigstens einen über die elektrisch isolierende Oberfläche des Fügeabschnittes erhabenen Elektrodenkörpers bietet sich die sog. Dickschichttechnologie an, mit der Schichtablagerungen mit Schichtdicken im µm-Bereich oder darüber erzeugbar sind. Alternativ ist es möglich, die erhabenen Elektrodenkörperstrukturen im Wege eines ganzflächigen Aufbringens einer elektrisch leitfähigen Folie oder Paste auf die elektrisch isolierende Oberfläche des Fügeabschnittes herzustellen, die nachträglich vorzugsweise mittels Laserstrahlung strukturiert wird. Desweitere eignen sich galvanische Abscheidungstechniken zur Ausbildung der Elektrodenkörperstrukturen.

Der lösungsgemäß ausgebildete Steckverbinder eignet sich insbesondere für die Kontaktierung einer Vielzahl am Stecker- und Buchsenteil vorgesehener Elektroden- und Gegenelektrodenkörper, um eine möglichst große Anzahl von elektrischen Übertragungskanälen mit Hilfe des Steckverbinders zu kontaktieren. So sind die Stecker-seitigen Elektrodenkörper jeweils verteilt an der wenigstens einen elektrisch isolierenden Oberfläche angeordnet, die beim Einführen des Steckerteils in das Buchsenteil parallel oder im Wesentlichen parallel zur Einschubrichtung orientiert ist. Auf dieser Weise kann durch entsprechende Wahl von Form und Größe dieser Oberfläche genügend viel Platz für die Unterbringung einer großen Vielzahl von Elektrodenkörper geschaffen werden, ohne dabei den Durchmesser des implantierbaren Steckverbinders zu erweitern, den es möglichst klein zu wählen gilt. In geeigneter Weise sind auch die an dem wenigstens einen die Einschuböffnung bereichsweise seitlich begrenzenden, elektrisch isolierenden Wandabschnitt angebrachten Gegenelektrodenkörper auszubilden und anzubringen.

Um die Vielzahl der am Stecker- und Buchsenteil vorgesehenen Elektroden- und Gegenelektrodenkörper noch weiter zu steigern verfügt der Steckerteil vorzugsweise über zwei sich gegenüberliegende, voneinander abgewandt orientierte elektrisch isolierende Oberflächen, die vorzugsweise jeweils eine Vielzahl von Elektrodenkörpern jeweils mit einer freizugänglichen Elektrodenoberfläche umfassen. Ebenso umfasst das Buchsenteil innerhalb der einseitig offen ausgebildeten Einschuböffnung zwei sich gegenüberliegende, einander zugewandt orientierte, elektrisch isolierende Wandabschnitte, deren Oberflächen jeweils die gleiche Vielzahl von Gegenelektrodenkörpern mit jeweils einer frei zugänglichen Gegenelektrodenoberfläche umfassen. Die Buchsen-seitigen, elektrisch isolierenden Wandabschnitte sind jeweils einer der elektrisch isolierenden Oberfläche des Fügeabschnittes des Steckerteils im gefügten derart zugewandt orientiert, so dass sich die Gegenelektrodenoberflächen die Elektrodenoberflächen mittel- oder unmittelbar berühren. Auf diese Weise sind miniaturisierte Steckverbinder realisierbar, die bis zu 256 getrennte elektrische Übertragungskanäle, vorzugsweise bis zu 50 Übertragungskanäle miteinander zu verbinden vermögen.

Ferner ist es denkbar zusätzliche Elektrodenkörper an einen Seitenwandbereich anzubringen, der die beiden vorstehend erläuterten, jeweils voneinander abgewandt orientierten, elektrisch isolierenden Oberflächen verbindet.

Weitere Einzelheiten zur Ausgestaltung des lösungsgemäßen Steckverbinders sind der weiteren Beschreibung unter Bezugnahme auf die Ausführungsbeispiele zu entnehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Schematisierte, perspektivische Ansicht auf ein Steckerteil,
- Fig. 2a, b: Längsschnittansicht sowie perspektivische Ansicht eines Buchsenteils,
- Fig. 3a,b,c: Längsschnittdarstellungen durch alternative Steckerteile,
- Fig. 4: Längsschnittdarstellung eines in ein Buchsenteil gefügtes Steckerteil,
- Fig. 5a - d: Draufsichtdarstellungen auf Stecker- und Buchsenteil zur Illustration des Einsteckvorganges.

### Wege zur Ausführung der Erfindung, gewerbliche Verwendbarkeit

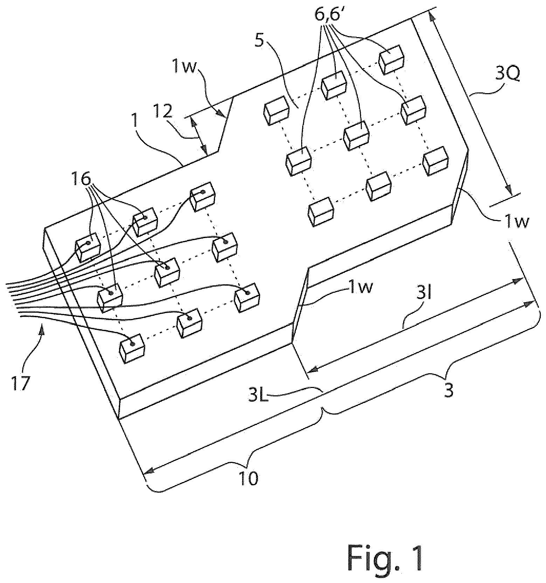

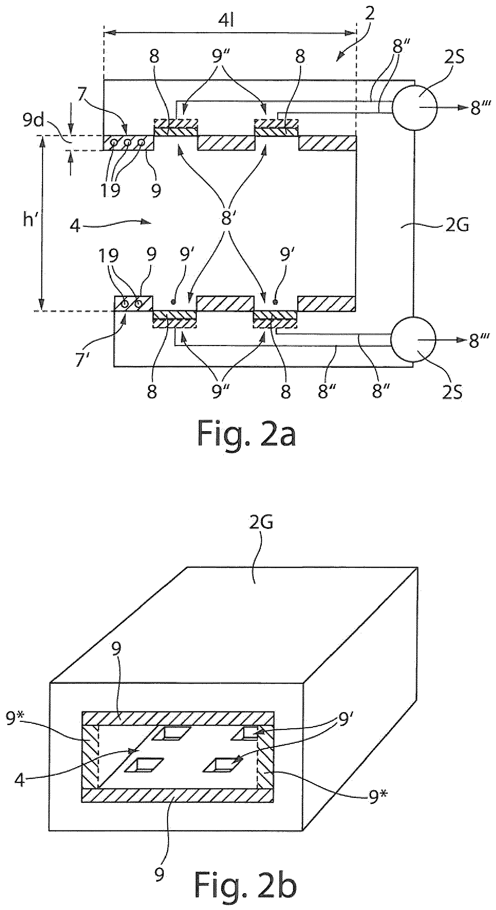

Figur 1 zeigt einen Steckerteil 1 in perspektivisch schematisierter Darstellung, der sich im Wesentlichen plattenförmig ausgebildet ist und über eine Längserstreckung 3L sowie eine Quererstreckung 3Q verfügt. Das Steckerteil 1 weist einen Fügeabschnitt 3 auf, der über eine Längserstreckung 3l verfügt und in ein in Figur 2 näher illustriertes Buchsenteil einführbar ist. Einstückig mit dem Fügeabschnitt 3 verbunden, sieht das Steckerteil 1 einen Steckerabschnitt 10 vor, der im gefügten Zustand innerhalb des in Figur 2 näher erläuterten Buchsenteils aus dem Buchsenteil 2 hinausragt. Selbstverständlich sind Bauabwandlungen dergestalt denkbar, bei denen der Steckerteil über seine gesamte Steckerlänge innerhalb des Buchsenteils Platz findet.

Das in Figur 1 dargestellte Steckerteil 1 verfügt über eine elektrisch isolierende Oberfläche 5, die sich einheitlich sowohl im Steckerabschnitt 10 als auch Fügeabschnitt 3 erstreckt.

Im Fügeabschnitt 3 des Steckerteils 1 sind im Ausführungsbeispiel neun Elektrodenkörper 6 angeordnet, die jeweils über eine obere Elektrodenoberfläche 6' verfügen. Entsprechend sind im Steckerabschnitt 10 gleichfalls neun elektrische Kontakte 16 vorgesehen, an denen bspw. über Lötverbindungen oder ähnliche elektrische Fügetechniken elektrische Zu- bzw. Ableitungen 17 befestigt sind. Die steckerabschnitt-seitig vorgesehenen elektrischen Kontakte 16 sind innerhalb des Steckerteils 1 in geeigneter Weise mit den im Fügeabschnitt 3 angeordneten Elektrodenkörper 6 elektrisch verbunden. Es sind Steckverbinder denkbar die 40, 50 und mehr Elektrodenkörper 6 an der Oberfläche 5 vorsehen können.

Das in Figur 1 dargestellte Steckerteil 1 zeichnet sich zudem auch durch einen seitlichen bzw. lateralen Versatz 12 aus, über den der Fügeabschnitt 3 seitlich zum Steckerabschnitt 10 angeordnet ist. Hierzu sind zwischen dem Fügeabschnitt 3 und dem Steckerabschnitt 10 zwei diagonal zur Längsrichtung 3L verlaufende Steckerteilseitenwände 1w vorgesehen. Eine entsprechend dimensionierte Seitenwand 1w sieht der Steckerteil 1 zusätzlich am stirnseitigen Ende des Fügeabschnittes 3 auf.

In Figur 2a ist ein Längsschnitt durch ein Buchsenteil 2 illustriert. In Figur 2b ist eine perspektivische Ansicht auf das Buchsenteil 2 dargestellt, das über eine Einschuböffnung 4 verfügt, in die der Fügeabschnitt 3 des Steckerteils 1 stirnseitig vollständig einschiebbar ist. Das Buchsenteil 2 weist ein formstabil, starres Gehäuse 2G auf und verfügt im illustrierten Ausführungsbeispiel über zwei sich gegenüberliegende, elektrisch isolierende Wandabschnitte 7, 7', an deren Oberflächen Gegenelektrodenkörper 8 mit jeweils frei zugänglichen Gegenelektrodenoberflächen 8' angeordnet sind. Die Gegenelektrodenkörper 8 sind über im Gehäuse 2G integrierte elektrische Verbindungsleitungen 8" und einer elektrischen Schnittstelle 2S mit einer vom Gehäuse 2G wegführenden elektrischen Leitung 8"' oder mehreren Leitungen 8"' verbunden.

Fest mit den elektrisch isolierenden Wandabschnitten 7 verbunden weist das Buchsenteil 2 jeweils eine elektrisch isolierende Polymerschicht 9 auf, die über eine Polymerschichtdicke 9d verfügt und Ausnehmungen 9' aufweist, die in Form und Größe jeweils an die Gegenelektrodenoberflächen 8' angepasst sind, so dass die Polymerschichten 9 die Gegenelektrodenoberflächen 8' vorzugsweise bündig und fluiddicht peripher umgeben.
Optional ist jeweils unterhalb jedes Gegenelektrodenkörpers 8, d.h. zwischen dem formstabilen, steifen Gehäuse 2G und jedem Gegenelektrodenkörper 8 ebenfalls eine Polymerschicht 9" eingebracht. Auf diese Weise ist die Lagerung des Gegenelektrodenkörpers 8 elastisch relativ zum Gehäuse 2G, was sich vorteilhaft im Falle einer kraftbeaufschlagten Kontaktierung mit einem entsprechenden Elektrodenkörper 6 auswirkt.

Die Ausgestaltungen des Buchsen- und Steckerteils 2, 1 sind entsprechend aneinander angepasst, das heißt die Anzahl und Anordnung der im Buchsenteil 2 vorgesehenen Gegenelektrodenkörper 8 entspricht der Anzahl und Anordnung der im Steckerteil 1 angebrachten Elektrodenkörper 6. Auch die Form und Dimensionierung der Einschuböffnung 4 innerhalb des Buchsenteils 2 sind unter Maßgabe von Form und Größe des Fügeabschnittes 3 des Steckerteils 1 entsprechend aufeinander abgestimmt. Im Falle des in Figur 2 illustrierten Buchsenteils 2 würde ein geeignet ausgestaltetes Steckerteil 1 über jeweils vier Elektrodenkörper jeweils an der Ober- und Unterseite des Fügeabschnittes 3 verfügen.

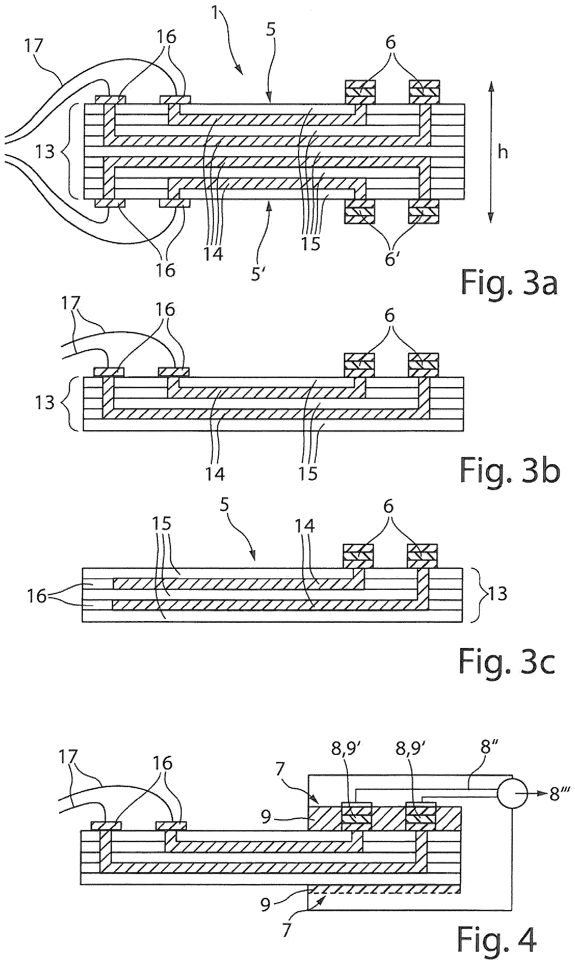

Ein Längsschnitt durch einen Steckerteil 1 mit jeweils vier an der Ober- und Unterseite des Fügeabschnittes 3 angebrachten Elektrodenkörpern 6 ist in Figur 3a illustriert. Der Steckerteil 3 weist einen Stapelschichtverbund 13 mit einer Abfolge elektrisch leitender Schichtbereiche 14 und elektrisch isolierender Schichtbereiche 15 auf. Zur Herstellung des Stapelschichtverbundes 13 eignen sich Multilayer-Prozesse, die die Herstellung so genannter Niedertemperatur-Einbrandkeramiken, kurz LTCC, oder Hochtemperatur-Mehrlagenkeramiken, kurz HTCC, erlauben. Die elektrischen Schichtbereiche 14 sind vorzugsweise aus Gold, Platin oder Iridium gefertigt, wohingegen die elektrisch isolierenden Schichtbereiche 15 vorzugsweise aus keramischen Materialien bestehen.

Der in Figur 3a illustrierte Steckverbinder 1 sieht sowohl an der Ober- als auch Unterseite Elektrodenkörper 6 sowie elektrische Kontakte 16 vor. Die elektrischen Kontakte 16 dienen für die Ausbildung vorzugsweise von Lötstellen zum Anschluss von elektrischen Verbindungskabeln 17. Vorzugsweise werden die elektrischen Kontakte 16 sowie die Elektrodenkörper 6 mittels Dickschichttechnologie, beispielsweise im Wege eines Siebdruckes, hergestellt. Ebenso bieten sich hierzu Folien- oder Lackschichtapplikationen auf den Oberflächen 5, 5' an. Die applizierte Lackschicht oder Folie wird die im Weiteren, beispielsweise mittels Laserstrahlung, nachbearbeitet und strukturiert.

Ferner ist es denkbar den in Figur 3a dargestellten Steckverbinder 1 mit wenigstens einem zusätzlichen Elektrodenkörper zu versehen, der längs einer Seitenkante des Stapelschichtverbundes 13 angeordnet ist, um auf dieser Weise die Anzahl der Elektrodenkörper weiter zu erhöhen.

Die Dimensionierung des Steckerteils 1 erfolgt in Abstimmung mit der Dimensionierung des Buchsenteils 2. Die Gesamtdicke h des Steckerteils 1 im Fügebereich 3 einschließlich der jeweils über die Oberflächen 5, 5' erhabenen Elektrodenkörper 6 entspricht mit einem geringen Untermaß der maximalen Öffnungsbreite h' der Einschuböffnung 4 innerhalb des Buchsenteils 2. So werden die Polymerschichten 9 beim Einführen des Steckerteils 1 in das in Figur 2a, b dargestellte Buchsenteil 2 komprimiert und bei Erreichen der Endposition des Steckerteils 1 innerhalb des Buchsenteils 2 münden die Elektrodenkörper 6 in die Ausnehmungen 9' der Polymerschichten 9, wobei die Elektrodenoberflächen 6 sowie die jeweils gegenüberliegend angeordneten Gegenelektrodenoberflächen 8' in einem innigen elektrischen Flächenkontakt treten. In der Endposition umschließen die Polymerschichten 9 die Elektrodenkörper 6 vollumfänglich. Darüber hinaus schmiegen sich die Polymerschichten 9 kompressionskraftbeaufschlagt an die Oberflächen 5 innerhalb des Fügeabschnittes 3 des Steckerteils 1 an. Ein Eindringen von Feuchtigkeit in den Bereich der Elektrodenkörper 6 ist somit zumindest längs der Grenzfläche zwischen Ober- und Unterseite des Steckerteils 1 und den Polymerschichten 9 auszuschließen.

Um die Sperrwirkung gegen Wasser- bzw. Feuchtigkeitseintritt längs der Seitenwände von Stecker- 1 und Buchsenteil 2 zu erhöhen, können optional zusätzliche Polymerschichten 9* vorzugsweise Buchsenteil-seitig an den Seitenwandbereichen innerhalb der Einschuböffnung 4 angebracht werden, siehe strichlierte Polymerschichten 9* in Figur 2b.

Ferner können die Polymerschichten 9 oder 9* zur weiteren Verbesserung der Sperrwirkung zumindest bereichsweise mit hygroskopischen Materialbestandteilen 19 kombiniert werden, die in Gegenwart von Feuchtigkeit Wasser binden und somit die Polymerschicht 9, 9* aufquellen lassen, siehe Figur 2a, b. Durch die aufquellende Volumenvergrößerung der Polymerschicht 9 erhöht sich unmittelbar die auf die Polymerschicht 9 einwirkende Kompressionskraft und die damit verbundene Sperrwirkung bzw. Dichtfunktion gegenüber Feuchtigkeits- bzw. Wassereintritt. Vorzugsweise sind die hygroskopischen Materialbestandteile 19 in Bereichen der Polymerschicht 9, 9* nahe dem Öffnungsbereich der Einschuböffnung 4 vorgesehen. Aus Gründen einer möglichst einheitlichen Sperrwirkung kann die gesamte Polymerschicht 9 mit hygroskopischen Materialbestandteilen durchmischt bzw. angereichert sein.

Eine weitere alternative Ausgestaltung für die Ausbildung des Steckerteils 1 ist in Figur 3b illustriert, der im Unterschied zu dem in 3a gezeigten Steckverbinder lediglich an der Oberseite 5 des Stapelschichtverbundes 13 elektrische Kontakte 16 und Elektrodenkörper 6 aufweist. Alternativ zur Ausbildung elektrischer Kontaktstellen 16 auf der Oberfläche 5 innerhalb des Steckerabschnittes 10 sind die elektrischen Kontakte 16 in dem Figur 3c illustrierten Ausführungsbeispiel als Steckkontaktbuchsen in lateraler Erstreckung zu den einzelnen elektrischen Schichtbereichen 14 ausgebildet.

Figur 4 zeigt eine Längsschnittdarstellung des in Figur 3b illustrierten Steckverbinders 1 innerhalb eines entsprechend konfektionierten Buchsenteils 2. Die Elektrodenkörper 6 des Steckverbinders 1 befinden sich innerhalb der Ausnehmungen 9' der Polymerschicht 9, die am oberen elektrisch isolierenden Wandabschnitt 7 fest angebracht ist. Optional kann auch eine weitere Polymerschicht 9 am unteren elektrisch isolierenden Wandabschnitt 7 angebracht sein. Ein möglicher Feuchtigkeitseintritt in den Zwischenspalt zwischen der elektrisch isolierten Oberfläche 5 des Steckerteils 1 und der Polymerschicht 9 kann ausgeschlossen werden, zumal die Polymerschicht aufgrund entsprechender Eigendickenwahl einer zusätzlichen Kompressionskraft und einer damit verbundenen die Anpresskraft auf die Oberfläche 5 des Steckerteils 1 einer damit verbundenen Erhöhung der Dichtwirkung gepresst wird. Im illustrierten gefügten Zustand befinden sich die Elektroden- und Gegenelektrodenoberflächen in innigem elektrischen Flächenkontakt, so dass der Steckverbinder eine elektrische Verbindung zwischen den Kabeln 17 und 8''' herstellt.

Sämtliche Elektroden- und Gegenelektrodenkörper 6, 8 sowie die damit verbundenen Elektroden- und Gegenelektrodenoberflächen 6', 8' können beliebige Raumformen annehmen. So eignen sich insbesondere n-eckige, ovale, runde Umfangsränder für die Ausgestaltung der Elektroden- und Gegenelektrodenoberflächen 6', 8'.

Vorzugsweise sind die frei zugänglichen Elektrodenoberflächen (6') der Elektrodenkörper (6) parallel oder schräg zu der wenigstens einen elektrisch isolierenden Oberfläche (5, 5') orientiert, gleichsam sind die frei zugänglichen Gegenelektrodenoberflächen (8') der Gegenelektrodenkörper (8) parallel oder schräg zum elektrisch isolierenden Wandabschnitt (7, 7') orientiert.

In Kombination oder alternativ hierzu sind die Flächennormalen der elektrisch isolierenden Oberflächen (5, 5') sowie der elektrisch isolierenden Wandabschnitte (7, 7') jeweils orthogonal oder schräg zur Einschubrichtung orientiert, längs der der Fügeabschnitt des Steckerteils in die Einschuböffnung (4) des Buchsenteils (2) führbar ist.

Unter einer jeweils "schrägen Orientierung" im vorstehenden Sinne soll abweichend von einer mathematisch streng definierten Parallelität oder Orthogonalität eine Winkeltoleranz α von maximal α = ± 30° verstanden werden.

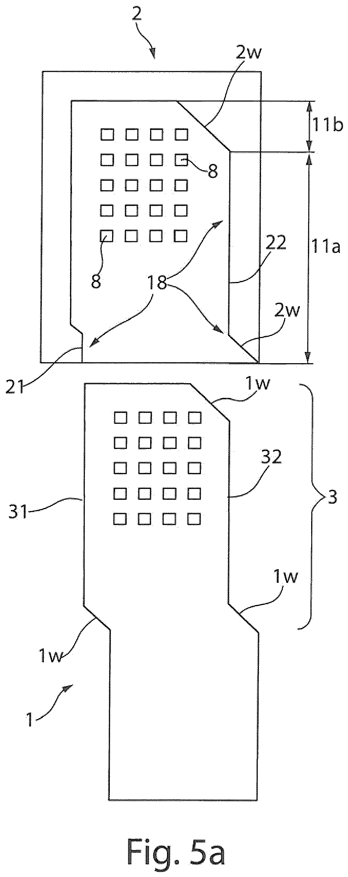

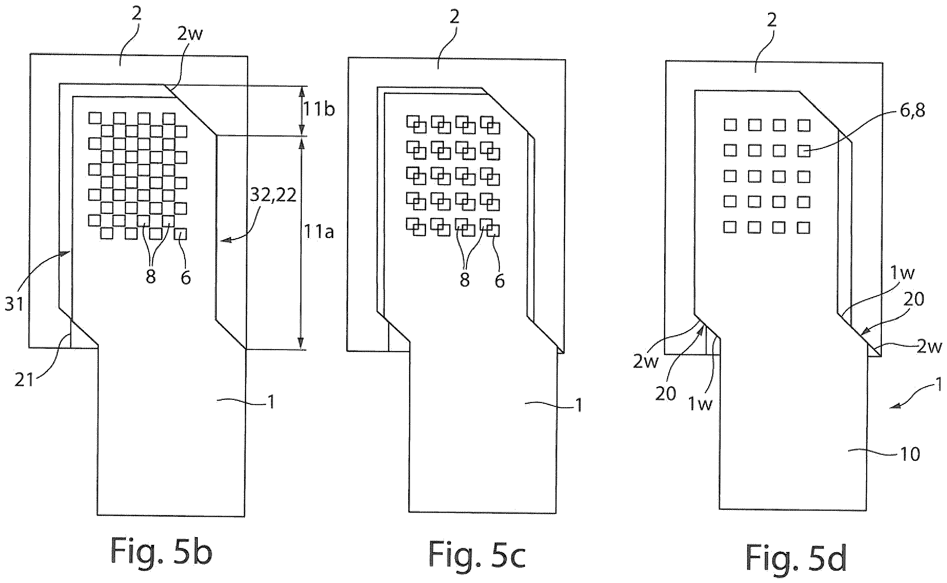

Die Einschubrichtung ist durch die Form und Ausgestaltung von Stecker- und Buchsenteil vorgegeben und ist, wie die weiteren Ausführungen zu den Figuren 5 a bis 5d zeigen, zunächst längs einer ersten Raumachse orientiert. Nachfolgend wird das Steckerteil zusätzlich längs und quer zur ersten Raumrichtung bewegt.

In Figur 5a ist die Draufsicht auf den Steckerteil 1 und Buchsenteil 2 dargestellt, wobei in der Darstellung des Buchsenteils 2 zusätzlich die Gegenelektrodenkörper 8 illustriert sind. Der Steckerteil 1 verfügt im gezeigten Ausführungsbeispiel im Fügeabschnitt 3 zwanzig an der Oberfläche 5, in Art eines Schachbrettmusters, separat angebrachte Elektrodenkörper 6. Demgegenüber weist das Buchsenteil 2 gleich viel gleichartig und gleichförmig angeordnete Gegenelektrodenkörper 8 auf. Das Einführen des Steckerteils 1 in die Einschuböffnung 4 des Buchsenteils 2 erfolgt längs einer Einschubkulisse 18, die in Einschubrichtung einen ersten Abschnitt 11 sowie einen nachfolgenden zweiten Abschnitt 12 aufweist.

Figur 5b zeigt einen Fügezustand zwischen Steckerteil 1 und Buchsenteil 2, bei dem das Steckerteil 1 längs des ersten Abschnittes 11 in das Buchsenteil 2 eingeführt ist. Hierzu gleiten die Seitenwände 31, 32 des Fügeabschnittes 3, siehe Figur 5a, zwangsgeführt längs der seitlichen Begrenzungswände 21, 22 innerhalb der Einschuböffnung 4 des Buchsenteils 2. Während des Einführvorganges ist somit die Relativlage des Steckerteils 1 zum Buchsenteil 2 exakt definiert. In eben dieser Einführkonstellation sind die vorzugsweise schachbrettartig angeordneten Elektrodenkörper 6 des Steckerteils 1 seitlich versetzt zu den ebenfalls schachbrettartig angeordneten Gegenelektrodenkörper 8 des Buchsenteils 2 geführt.

Hierdurch wird eine gegenseitige Berührung der Elektroden- und Gegenelektrodenkörper 6, 8 während des Einführvorganges bis zu der in Figur 5b dargestellten Konstellation vermieden.

Sobald die in Figur 1 erläuterten diagonal orientierten Steckerteil-seitigen Seitenwandflächen 1w die ebenfalls relativ zur Einschubrichtung diagonal verlaufenden Seitenwandflächen 2w des Buchsenteils 2 berühren, gelangen die Elektroden- und Gegenelektrodenflächen 6', 8' jeweils paarweise in zunehmende Überlappung, siehe Fig. 5c.

Nach Erreichen der Endposition des Steckerteils 1 innerhalb des Buchsenteils 2, siehe Figur 5d, überlappen die Elektroden- und Gegenelektrodenflächen 6', 8' jeweils vollständig.

Zum Zwecke einer Sicherung bzw. Schutzes gegen ein unkontrolliertes Herausrutschen des Steckerteils aus dem Buchsenteil kann in Einschubrichtung an der stirnseitigen Seitenkante des Steckerteils ein stegartig, strukturierte Fortsatz angebracht sein, der in Rasteingriff mit einer entsprechenden Buchsenteil-seitig angebrachten Ausnehmung gebracht werden kann und dafür sorgt, dass ein Lösen des Steckerteils vom Buchsenteil erst nach einem gezielten Vor- und/oder Querschieben des Steckerteils relativ zur Einschubrichtung und/oder erst durch eine definierte, senkrecht auf den strukturierten Fortsatz einwirkende Kraft möglich wird.

Durch den konstruktiv vorgegebenen seitlichen Versatz 12 zwischen Fügeabschnitt 3 und Steckerabschnitt 10, fügen sich die dem Steckerabschnitt 10 nächstliegenden Seitenwandabschnitte 1w an die entsprechend Buchsenteil-seitigen Seitenwandabschnitte 2w und bilden jeweils fluiddichte Dichtflächen 20 aus. Mit dieser konstruktiven Maßnahme können auf extra an den Seitenwänden des Stecker- und/oder des Buchsenteils anzubringende Polymerschichten, wie dies in Verbindung mit dem in Figur 2b illustrierten Ausführungsbeispiel exemplarisch gezeigt ist, verzichtet werden.

Nicht notwendigerweise ist die Form des Stecker- und Buchsenteils 1, 2 auf die in den vorstehenden Ausführungsbeispielen gezeigten Raumformen beschränkt. Auf diese Weise ist für eine allseitige fluiddichte Abdichtung des Steckerteils 1 innerhalb des Buchsenteils 2 gesorgt.

### Bezugszeichenliste

- 1: Steckerteil
- 1w: Diagonal verlaufende Seitenwandflanken
- 2: Buchsenteil
- 21: Buchsenteil-seitiger Seiteninnenwand
- 22: Buchsenteil-seitiger Seiteninnenwand
- 2w: Buchsenteil-seitiger diagonaler Wandabschnitt
- 3: Fügeabschnitt
- 31, 32: Seitenwand des Fügeabschnittes
- 3L: Längserstreckung des Steckerteils
- 3l: Längserstreckung des Fügeabschnittes
- 3Q: Quererstreckung des Fügeabschnittes
- 4: Einschuböffnung
- 4l: Längserstreckung der Einschuböffnung
- 5, 5': Elektrisch isolierende Oberfläche des Steckerteils
- 6: Elektrodenkörper
- 6': Elektrodenoberfläche
- 7: Elektrisch isolierender Wandabschnitt
- 8: Gegenelektrodenkörper
- 8': Gegenelektrodenoberfläche
- 8": Elektrische Verbindungsstruktur
- 8''': Elektrische Zu- bzw. Ableitung
- 9: Polymerschicht
- 9': Ausnehmung
- 9": Polymerschicht
- 9*: Polymerschicht an Seitenwand
- 10: Steckerabschnitt
- 11a: Erster Abschnitt
- 11b: Zweiter Abschnitt
- 12: Seitlicher Versatz
- 13: Stapelschichtverbund
- 14: Elektrische Schichtbereiche
- 15: Elektrisch isolierende Schichtbereiche
- 16: Elektrische Kontakte
- 16': Steckkontaktbuchse
- 17: Elektrische Zu- bzw. Ableitungen
- 18: Einschubkulisse
- 19: Hygroskopisches Material
- 20: Fluiddichte Dichtfläche
- h: Dicke des Steckerteils
- h': Öffnungsbreite
- 2G: Gehäuse
- 2S: elektrische Schnittstelle

## Patentansprüche

1. Implantierbarer, elektromechanischer Steckverbinder mit einem Stecker- (1) und einem Buchsenteil (2), von denen der Steckerteil (1) wenigstens einen Fügeabschnitt (3) aufweist, der vollständig, in eine einseitig offen ausgebildete Einschuböffnung (4) innerhalb des Buchsenteils (2) einführbar ist und über wenigstens eine elektrisch isolierende Oberfläche (5) verfügt, die wenigstens einen Elektrodenkörper (6) mit einer frei zugänglichen Elektrodenoberfläche (6') aufweist, und von denen das Buchsenteil (2) innerhalb der einseitig offen ausgebildeten Einschuböffnung (4) einen die Einschuböffnung (4) zumindest bereichsweise seitlich begrenzenden elektrisch isolierenden Wandabschnitt (7) aufweist, deren Oberfläche wenigstens einen Gegenelektrodenkörper (8) mit einer frei zugänglichen Gegenelektrodenoberfläche (8') vorsieht, wobei der elektrisch isolierende Wandabschnitt (7) der elektrisch isolierenden Oberfläche (5) des Fügeabschnittes (3) des Steckerteils (1) im Zustand einer vollständigen Einführung des Steckerteil-seitigen Fügeabschnittes (3) in der Einschuböffnung (4), derart zugewandt orientiert ist, dass die Gegenelektrodenoberfläche (8') die Elektrodenoberfläche (6') berührt, wobei der wenigstens eine Elektrodenkörper (6) erhaben gegenüber der elektrisch isolierenden Oberfläche (5) des Fügeabschnittes (3) ausgebildet ist und/oder der wenigstens eine Gegenelektrodenkörper (8) erhaben gegenüber der Oberfläche (8') des elektrisch isolierenden Wandabschnittes (7) ausgebildet ist, und
wobei zumindest bereichsweise zwischen der elektrisch isolierenden Oberfläche (5) des Fügeabschnittes (3) und der Oberfläche des elektrisch isolierenden Wandabschnittes (7) wenigstens eine elektrisch isolierende Polymerschicht (9) angeordnet ist, die die sich berührenden Gegenelektrodenoberfläche (8') und Elektrodenoberfläche (6') vollständig umgibt,
**dadurch gekennzeichnet, dass** zumindest der Steckerteil (1) im Rahmen eines Multilayer-Prozesses hergestellt ist,
dass der Steckerteil (1) eine Vielzahl von elektrisch leitenden Schichten (14) jeweils elektrisch voneinander isoliert, in einem Stapelschichtverbund (13) vorsieht,
dass die einzelnen elektrisch leitenden Schichten (14) jeweils elektrisch isoliert voneinander an der wenigstens einen elektrisch isolierenden Oberfläche (5) jeweils unter Ausbildung eines Kontaktbereiches münden, wobei die Kontaktbereiche jeweils mit einem über die elektrisch isolierende Oberfläche (5) erhabenen Elektrodenkörper (6) kontaktiert sind.

2. Steckverbinder nach Anspruch 1,
**dadurch gekennzeichnet, dass** die wenigstens eine elektrisch isolierende Polymerschicht (9) fest am elektrisch isolierenden Wandabschnitt (7) innerhalb der Einschuböffnung (4) des Buchsenteils (2) und/oder fest an der elektrisch isolierenden Oberfläche (5) des Fügeabschnittes (3) des Steckerteils (1) gefügt ist.

3. Steckverbinder nach Anspruch 1 oder 2,
**dadurch gekennzeichnet, dass** die wenigstens eine elektrisch isolierende Polymerschicht (9) eine Schichtdicke besitzt, die wenigstens einer gegenüber der Oberfläche des elektrisch isolierenden Wandabschnittes (7) orthogonalen Erhabenheit des wenigstens einen Gegenelektrodenkörpers (8) oder wenigstens einer gegenüber der elektrisch isolierenden Oberfläche (5) des Fügeabschnittes (3) orthogonalen Erhabenheit des wenigstens einen Elektrodenkörpers (6) entspricht.

4. Steckverbinder nach einem der Ansprüche 1 bis 3,
**dadurch gekennzeichnet, dass** die Größe und Form des Fügeabschnittes (3) sowie die Größe und Form der Einschuböffnung (4) sowie die Größe und Form der elektrisch isolierenden Polymerschicht (9) derart aufeinander abgestimmt sind, dass im Zustand einer vollständigen Einführung des Steckerteil-seitigen Fügeabschnittes (3) in die Einschuböffnung (4) die wenigstens eine elektrisch isolierende Polymerschicht (9) einer Kompressionskraft ausgesetzt ist, die zwischen der Oberfläche des elektrisch isolierenden Wandabschnittes (7) und der elektrisch isolierenden Oberfläche (5) des Fügeabschnittes (3) wirkt.

5. Steckverbinder nach einem der Ansprüche 1 bis 3,
**dadurch gekennzeichnet, dass** die Größe und Form des Fügeabschnittes (3) sowie die Größe und Form der Einschuböffnung (4) sowie die Größe und Form der elektrisch isolierenden Polymerschicht (9) derart aufeinander abgestimmt sind, dass im Zustand einer vollständigen Einführung des Steckerteil-seitigen Fügeabschnittes (3) in die Einschuböffnung (4) die frei zugängliche Elektrodenoberfläche (6') des wenigstens einen Elektrodenkörpers (6) kompressionskraftbeaufschlagt an der Oberfläche des wenigstens einen Gegenelektrodenkörpers (8) anliegt.

6. Steckverbinder nach einem der Ansprüche 1 bis 5,
**dadurch gekennzeichnet, dass** dem Fügeabschnitt (3) des Steckerteils (1) eine laterale Längs- (3l) und Quererstreckung (3Q) zugeordnet ist,
dass die Längserstreckung (3) des Fügeabschnittes wenigstens einer der Einschuböffnung (4) zugeordneten Einführtiefe (4l) entspricht und
dass die Einschuböffnung (4) eine wandseitige Einschubkulisse aufweist, längs der der Fügeabschnitt (3) während des Einführens in die Einschuböffnung (4) zwangsgeführt entlanggleitet, und
dass die Einschubkulisse einen ersten Abschnitt (11a) aufweist, längs dem der Fügeabschnitt (3) ausschließlich in dessen Längserstreckung (3l), sowie einen daran anschließenden zweiten Abschnitt (11b) aufweist, längs dem der Fügeabschnitt (3) in dessen Längs- (3l) und Quererstreckung (3Q) in die Einschuböffnung (4) einführbar ist.

7. Steckverbinder nach Anspruch 6,
**dadurch gekennzeichnet, dass** der wenigstens eine Steckerteil-seitige Elektrodenkörper (6) und der wenigstens eine Buchstenteil-seitige Gegenelektrodenkörper (8) derart angeordnet sind, dass die Elektroden- (6') und Gegenelektrodenoberfläche (8') einen in Quererstreckung (3Q) des Fügeabschnittes (3) orientierten Abstand (12) aufweisen, solange der Fügeabschnitt (3) längs des ersten Abschnittes (11a) entlanggleitet.

8. Steckverbinder nach Anspruch 7,
**dadurch gekennzeichnet, dass** der in Quererstreckung (3Q) des Fügeabschnittes (3) orientierte Abstand (12) zwischen der wenigstens einen Elektroden- und Gegenelektrodenoberfläche (6', 8') einem Maß entspricht, um das der Fügeabschnitt (3) nach Entlanggleiten längs des zweiten Abschnittes (11) in dessen Quererstreckung (3Q) innerhalb der Einschuböffnung (4) auslenkbar ist.

9. Steckverbinder nach einem der Ansprüche 1 bis 8,
**dadurch gekennzeichnet, dass** der Multilayer-Prozess Niedertemperatur-Einbrand-Keramiken, kurz LTCC, oder Hochtemperatur-Mehrlagenkeramik, kurz HTCC nutzt, und dass der wenigstens eine über die elektrisch isolierende Oberfläche (5) erhabene Elektrodenkörper (6) im Wege einer Dickschichttechnologie herstellbar ist.

10. Steckverbinder nach einem der Ansprüche 1 bis 9,
**dadurch gekennzeichnet, dass** der Steckerteil (1) einen sich an den Fügeabschnitt (3) anschließenden Steckerabschnitt (10) aufweist, an dem wenigstens ein elektrischer Kontakt (16) vorgesehen ist, der elektrisch mit der wenigstens einen im Fügeabschnitt (3) vorgesehenen Elektrodenoberfläche (6') verbunden ist.

11. Steckverbinder nach einem der Ansprüche 1 bis 10,
**dadurch gekennzeichnet, dass** die wenigstens eine elektrisch isolierende Polymerschicht (9) zumindest Schichtbereiche mit hygroskopischen Eigenschaften umfasst.

12. Steckverbinder nach Anspruch 11,
**dadurch gekennzeichnet, dass** die Schichtbereiche mit hygroskopischen Eigenschaften zumindest abschnittweise derart relativ zu der wenigstens einen Elektrodenoberfläche (6') und/oder Gegenelektrodenoberfläche (8') angeordnet sind, dass im Zustand einer vollständigen Einführung des Steckerteil-seitigen Fügeabschnittes (3) in die Einschuböffnung (4) eine feuchtigkeitsbedingte Quellung der hygroskopischen Schichtbereiche zu einer Erhöhung der zwischen der Oberfläche des elektrisch isolierenden Wandabschnittes (7) und der elektrisch isolierenden Oberfläche (5) des Fügeabschnittes (3) wirkenden Kompressionskraft führt.

13. Steckverbinder nach einem der Ansprüche 1 bis 12,
**dadurch gekennzeichnet, dass** der Steckerteil (1) über zwei sich gegenüberliegende, voneinander abgewandt orientierte elektrisch isolierende Oberflächen (5, 5') verfügt, die jeweils wenigstens einen Elektrodenkörper (6) mit einer frei zugänglichen Elektrodenoberfläche (6') aufweisen, und
dass das Buchsenteil (2) innerhalb der einseitig offen ausgebildeten Einschuböffnung (4) zwei sich seitlich gegenüberliegende, einander zugewandt orientierte, elektrisch isolierende Wandabschnitte (7, 7') aufweist, deren Oberfläche jeweils wenigstens einen Gegenelektrodenkörper (8) mit einer frei zugänglichen Gegenelektrodenoberfläche (8') vorsieht, wobei die elektrisch isolierenden Wandabschnitte (7, 7') jeweils einer der elektrisch isolierenden Oberflächen (5, 5') des Fügeabschnittes (3) des Steckerteils (1) im Zustand einer vollständigen Einführung des Steckerteil-seitigen Fügeabschnittes (3) in der Einschuböffnung (4), derart zugewandt orientiert sind, dass die Gegenelektrodenoberflächen (8') die Elektrodenoberflächen (6') jeweils berühren.

14. Steckverbindern nach einem der Ansprüche 1 bis 13,
**dadurch gekennzeichnet, dass** der elektrisch isolierenden Oberfläche (5, 5') sowie dem elektrisch isolierenden Wandabschnitt (7, 7') jeweils eine die Flächennormale zugeordnet ist, die orthogonal oder schräg zur Einschubrichtung orientiert sind, längs der der Fügeabschnitt (3) des Steckerteils (1) in die Einschuböffnung (4) des Buchsenteils (2) führbar ist.

15. Steckverbinder nach einem der Ansprüche 1 bis 14,
**dadurch gekennzeichnet, dass** die frei zugängliche Elektrodenoberfläche (6') des wenigstens einen Elektrodenkörpers (6) parallel oder schräg zu der wenigstens einen elektrisch isolierenden Oberfläche (5, 5') orientiert ist, und
dass die frei zugängliche Gegenelektrodenoberfläche (8') des wenigstens einen Gegenelektrodenkörpers (8) parallel oder schräg zu dem elektrisch isolierenden Wandabschnitt (7, 7') orientiert ist.

## Claims

1. Implantable, electromechanical plug connector with a plug part (1) and a socket part (2), of which the plug part (1) comprises at least one joining portion (3) which can be fully inserted into an insertion opening (4) which is open on one side and formed within the socket part (2) and comprises at least one electrically insulating surface (5) which has at least one electrode body (6) with a freely accessible electrode surface (6') and of which the socket part (2) within the insertion opening (4) which is open on one side has an electrically insulating wall section (7), at least in sections delimiting the insertion opening (4), the surface of which provides at least one counter-electrode body (8) with a freely accessible counter-electrode surface (8'), wherein the electrically insulating wall section (7) is oriented towards the electrically insulating surface (5) of the joining portion (3) of the plug part (1) in the state of full insertion of the joining portion (3) into the insertion opening (4) in such a way that the counter-electrode surface (8') contacts the electrode surface (6') wherein the at least one electrode body (6) is raised with regard to the electrically insulating surface (5) of the joining portion (3) and/or the at least one counter-electrode body (8) is raised with regard to the surface (8') of the electrically insulating wall section (7) and wherein at least in sections between the electrically insulating surface (5) of the joining portion (3) and the surface of the electrically insulating wall section (7) at least one electrically insulating polymer layer (9) is arranged which completely surrounds the touching counter-electrode surface (8') and electrode surface (6')
**characterised in that** at least the plug part (1) is manufactured as part of a multilayer process,
**in that** the plug part (1) envisages a plurality of electrically conducting layers (14), each electrically insulated from each other in a stacked layer composite (13), **in that** the individual electrically conducting layers (14), each electrically insulated from each other, each open onto the at least one electrically insulating surface (5) forming a contact area, wherein the contact areas are each in contact with an electrode body (6) raised above the electrically insulating surface (5).

2. Plug connector according to claim 1
**characterised in that** the at least one electrically insulating polymer layer (9) is firmly joined to the electrically insulating wall section (7) within the insertion opening (4) of the socket part (2) and/or firmly joined to the electrically insulating surface (5) of the joining portion (3) of the plug part (1).

3. Plug connector according to claim 1 or 2
**characterised in that** the at least one electrically insulating polymer layer (9) has a layer thickness which corresponds with at least one orthogonal elevation of the at least one counter-electrode body (8) in relation to the surface of the electrically insulating wall section (7) or at least one orthogonal elevation of the at least one electrode body (6) in relation to the electrically insulating surface (5) of the joining portion (3).

4. Plug connector according to any one of claims 1 to 3
**characterised in that** the size and shape of the joining portion (3) as well as the size and shape of the insertion opening (4) as well as the size and shape of the electrically insulating polymer layer (9) are matched to each other in such a way that in the state of full insertion of the joining portion (3) on the plug side into the insertion opening (4), the at least one electrically insulating polymer layer (9) is subjected to a compression force which acts between the surface of the electrically insulating wall section (7) and the electrically insulating surface (5) of the joining portion (3).

5. Plug connector according to any one of claims 1 to 3
**characterised in that** the size and shape of the joining portion (3) as well as the size and shape of the insertion opening (4) as well as the size and shape of the electrically insulating polymer layer (9) are matched to each other in such a way that in the state of complete insertion of the joining portion (3) on the plug side into the insertion opening (4) the freely accessible electrode surface (6') of the at least one electrode body (6) adjoins the surface of the at least one counter-electrode body (8) with a compression force exerted on it.

6. Plug connector according to any one of claims 1 to 5
**characterised in that** a lateral longitudinal (3l) and transverse (3Q) extent is assigned to the joining portion (3) of the plug part (1),
**in that** the longitudinal extent (3) of the joining portion at least corresponds with an insertion depth (41) assigned to the insertion opening (4) and
**in that** the insertion opening (4) has a wall-side insertion guide along which the joining portion (3) slide is forced to slide during insertion into the insertion opening (4) and
**in that** the insertion guide comprises a first section (11a) along which the joining portion (3) can be inserted exclusively in its longitudinal direction (3l), and an adjoining second section (11b) along which the joining portion (3) can be inserted into the insertion opening (4) in its longitudinal (31) and transverse direction (3Q).

7. Plug connector according to claim 6
**characterised in that** the at least one plug-side electrode body (6) and the at least one socket-side counter-electrode body are arranged in such a way that the electrode surface (6') and counter-electrode surface (8') exhibit a distance (12) orientated in a transverse direction (3Q) of the joining portion (3) as long as the joining portion (3) is sliding along the first section (11a).

8. Plug connector according to claim 7
**characterised in that** the distance (12) orientated in the transverse direction (3Q) of the joining portion (3) corresponds to a measurement between the at least one electrode surface and counter-electrode surface (6', 8') by which the joining portion (3) is deflectable after sliding along the second section (11) in its transverse direction (3Q) within the insertion opening (4) .

9. Plug connector according to any one of claims 1 to 8
**characterised in that** the multi-layer process uses low-temperature co-fired ceramics, LTCC in short, or high-temperature co-fired ceramics, HTCC in short, and **in that** the at least one electrode body (6) raised above the electrically insulating surface (5) can be produced by way of thick-layer technology.

10. Plug connector according to any one of claims 1 to 9
**characterised in that** the plug part (1) comprises a plug section (10) adjoining the joining portion (3), on which at least one electrical contact (16) is provided which is electrically connected to the at least one electrode surface (6') provided in the joining surface (3) .

11. Plug connector according to any one of claims 1 to 10
**characterised in that** the at least one electrically insulating polymer layer (9) comprises at least one layer area with hygroscopic properties.

12. Plug connector according to claim 11
**characterised in that** the layer areas with hygroscopic properties are arranged at least in sections relative to the at least one electrode surface (6') and/or counter-electrode surface (8') in such a way that in the state of complete insertion of the plug-side joining portion (3) into the insertion opening (4), moisture-caused swelling of the hygroscopic layer areas leads to an increase in the compression force acting between the surface of the electrically insulating wall section (7) and the electrically insulating surface (5) of the joining portion (3).

13. Plug connector according to any one of claims 1 to 12
**characterised in that** the plug part (1) comprises two opposite electrically insulating surfaces (5, 5') orientated away from each other which each have at least one electrode body (6) with a freely accessible electrode surface (6') and
**in that** the socket part (2), within the insertion opening (4) open on one side, comprises two opposite electrically insulating wall surfaces (7, 7') orientated towards each other, the surface of each of which provides at least one counter-electrode body (8) with a freely accessible counter-electrode surface (8'), wherein the electrically insulating wall sections (7, 7') of each of the electrically insulating surfaces (5, 5') of the joining portion (3) of the plug part (1) in the state of complete insertion of the plug-side joining portion (3) into the insertion opening (3) are oriented towards each other in such a way that the counter-electrode surfaces (8') each contact the electrode surfaces (6').

14. Plug connector according to any one of claims 1 to 13
**characterised in that** the electrically insulating surface (5, 5') as well as the electrically insulating wall section (7, 7') is each assigned a surface normal, orthogonally or obliquely orientated to the insertion direction, along which the joining portion (3) of the plug part (1) can be guided into the insertion opening (4) of the socket part (2).

15. Plug connector according to any one of claims 1 to 14
**characterised in that** the freely accessible electrode surface (6') of the at least one electrode body (6) is orientated in parallel or obliquely to the at least one electrically insulating surface (5, 5') and
**in that** the freely accessible counter-electrode surface (8') of the at least one counter-electrode body (8) is orientated in parallel or obliquely to the electrically insulating wall section (7, 7').

## Revendications

1. Connecteur électromécanique implantable avec une partie mâle (1) et une partie femelle (2), dont la partie mâle (1) comporte au moins une section d'assemblage (3), qui peut être complètement introduite dans une ouverture d'introduction (4) constituée ouverte d'un côté à l'intérieur de la partie femelle (2) et dispose d'au moins une surface (5) électriquement isolante, qui comporte au moins un corps d'électrode (6) avec une surface d'électrode (6') librement accessible et dont la partie femelle (2) à l'intérieur de l'ouverture d'introduction (4) constituée ouverte d'un côté comporte une section de paroi (7) électriquement isolante délimitant latéralement au moins par endroits l'ouverture d'introduction (4), dont la surface prévoit au moins un corps d'électrode opposé (8) avec une surface d'électrode opposée (8') librement accessible, sachant que la section de paroi (7) électriquement isolante de la surface (5) électriquement isolante de la section d'assemblage (3) de la partie mâle (1) est orientée à l'état d'une introduction complète de la section d'assemblage (3) côté partie mâle dans l'ouverture d'introduction (4) tournée de telle manière que la surface d'électrode opposée (8') touche la surface d'électrode (6'), sachant qu'au moins un corps d'électrode (6) est constitué en saillie par rapport à la surface (5) électriquement isolante (8') de la section d'assemblage (3) et/ou au moins un corps d'électrode opposé (8) est constitué en saillie par rapport à la surface (8') de la paroi (7) électriquement isolante et sachant qu'au moins par endroit entre la surface (5) électriquement isolante de la section d'assemblage (3) et la surface de la section de paroi (7) électriquement isolante est disposée au moins une couche de polymère (9) électriquement isolante, qui entoure complètement la surface d'électrode opposée (8') et la surface d'électrode (6') se touchant,
**caractérisé en ce qu'**au moins la partie mâle (1) est fabriquée dans le cadre d'un processus multicouches,
**en ce que** la partie mâle (1) isole électriquement respectivement l'une de l'autre une pluralité de couches électro-conductrices (14), prévoit dans une combinaison de couches empilées (13) que les couches (14) individuelles électro-conductrices respectivement isolées électriquement l'une de l'autre débouchent sur au moins une surface (5) électriquement isolante en constituant respectivement une zone de contact , sachant que les zones de contact sont respectivement en contact avec un corps d'électrode (6) en saillie sur la surface (5) électriquement isolante.

2. Connecteur selon la revendication 1,
**caractérisé en ce qu'**au moins une couche de polymère (9) électriquement isolante est fermement assemblée à la section de paroi (7) électriquement isolante à l'intérieur de l'ouverture d'introduction (4) de la partie femelle (2) et/ou fermement assemblée à la surface (5) électriquement isolante de la section d'assemblage (3) de la partie mâle (1).

3. Connecteur selon la revendication 1 ou 2,
**caractérisé en ce qu'**au moins une couche de polymère (9) électriquement isolante possède une épaisseur de couche, qui correspond au moins à un relief saillant orthogonal d'au moins un corps d'électrode opposé (8) par rapport à la surface de la section de paroi (7) électriquement isolante ou au moins à un relief saillant orthogonal d'au moins un corps d'électrode (6) par rapport à la surface (5) électriquement isolante de la section d'assemblage (3).

4. Connecteur selon l'une quelconque des revendications 1 à 3,
**caractérisé en ce que** la taille et la forme de la section d'assemblage (3) ainsi que la taille et la forme de l'ouverture d'introduction (4) et la taille et la forme de la couche de polymère (9) électriquement isolante sont adaptées l'une à l'autre de telle manière qu'à l'état d'une introduction complète de la section d'assemblage (3) côté partie mâle dans l'ouverture d'introduction (4), au moins une couche de polymère (9) électriquement isolante est exposée à une force de compression qui agit entre la surface de la section de paroi (7) électriquement isolante et la surface (5) électriquement isolante de la section d'assemblage (3).

5. Connecteur selon l'une quelconque des revendications 1 à 3,
**caractérisé en ce que** la taille et la forme de la section d'assemblage (3) ainsi que la taille et la forme de l'ouverture d'introduction (4) et la taille et la forme de la couche de polymère (9) électriquement isolante sont adaptées l'une à l'autre de telle manière qu'à l'état d'une introduction complète de la section d'assemblage (3) côté partie mâle dans l'ouverture d'introduction (4), la surface d'électrode (6') librement accessible d'au moins un corps d'électrode (6) vient s'appliquer sollicitée par une force de compression à la surface d'au moins un corps d'électrode opposé (8).

6. Connecteur selon l'une quelconque des revendications 1 à 5,
**caractérisé en ce qu'**une extension longitudinale (3l) et transversale (3Q) latérale est attribuée à la section d'assemblage (3) de la partie mâle (1),
**en ce que** l'extension longitudinale (3) de la section d'assemblage correspond au moins à une profondeur
d'introduction (4l) attribuée à l'ouverture d'introduction (4), et
**en ce que** l'ouverture d'introduction (4) comporte une coulisse d'introduction côté paroi, le long de laquelle la section d'assemblage (3) glisse en guidage forcé pendant l'introduction dans l'ouverture d'introduction (4), et
**en ce que** la coulisse d'introduction comporte une première section (11a), le long de laquelle la section d'assemblage (3) peut être exclusivement introduite dans l'extension longitudinale (3l) de celle-ci, et comporte une deuxième section (11b) s'y raccordant, le long de laquelle la section d'assemblage (3) peut être introduite dans l'extension longitudinale (31) et transversale (3Q) de celle-ci dans l'ouverture d'introduction (4).

7. Connecteur selon la revendication 6,
**caractérisé en ce qu'**au moins un corps d'électrode (6) côté partie mâle et au moins un corps d'électrode opposé (8) côté partie femelle sont disposés de telle manière que la surface d'électrode (6') et la surface d'électrode opposée (8') comportent une distance (12) orientée dans l'extension transversale (3Q) de la section d'assemblage (3) tant que la section d'assemblage (3) glisse le long de la première section (11a).

8. Connecteur selon la revendication 7,
**caractérisé en ce que** la distance (12) orientée dans l'extension transversale (3Q) de la section d'assemblage (3) entre au moins une surface d'électrode et une surface d'électrode opposée (6', 8') correspond à une mesure à laquelle la section d'assemblage (3) peut être orientée après glissement le long de la deuxième section (11) dans l'extension transversale
(3Q) de celle-ci à l'intérieur de l'ouverture d'introduction (4).

9. Connecteur selon l'une quelconque des revendications 1 à 8,
**caractérisé en ce que** le processus multicouches utilise des céramiques cofrittées à basse température, en abrégé CCBT, ou des céramiques multicouches cofrittées à haute température, en abrégé, CCHT et **en ce qu'**au moins un corps d'électrode (6) en saillie sur la surface (5) électriquement isolante puisse être réalisé dans le cadre d'une technologie des couches épaisses.

10. Connecteur selon l'une quelconque des revendications 1 à 9,
**caractérisé en ce que** la partie mâle (1) comporte une section de connecteur (10) se raccordant à la section d'assemblage (3) sur laquelle est prévu au moins un contact électrique (16), qui est électriquement relié à au moins une surface d'électrode (6') prévue dans la section d'assemblage (3).

11. Connecteur selon l'une quelconque des revendications 1 à 10,
**caractérisé en ce qu'**au moins une couche de polymère (9) électriquement isolante comprend au moins des zones de couche avec des propriétés hygroscopiques.

12. Connecteur selon la revendication 11,
**caractérisé en ce que** les zones de couche avec des propriétés hygroscopiques sont disposées au moins par section par rapport à au moins une surface d'électrode (6') et/ou une surface d'électrode opposée (8') de telle manière qu'à l'état d'une introduction complète de la section d'assemblage (3) côté partie mâle dans l'ouverture d'introduction (4), un gonflement des zones de couche hygroscopiques dû à l'humidité conduit à une augmentation de la force de compression agissant entre la surface de la section de paroi (7) électriquement isolante et la surface (5) électriquement isolante de la section d'assemblage (3).

13. Connecteur selon l'une quelconque des revendications 1 à 12,
**caractérisé en ce que** la partie mâle (1) dispose de deux surfaces (5, 5') électriquement isolantes se superposant, orientées opposées l'une à l'autre, qui comportent respectivement au moins un corps d'électrode (6) avec une surface d'électrode (6') librement accessible et
**en ce que** la partie femelle (2) à l'intérieur de l'ouverture d'introduction (4) constituée ouverte d'un côté comporte deux sections de paroi (7, 7') électriquement isolantes, se superposant latéralement, orientées tournées l'une vers l'autre, dont la surface prévoit respectivement au moins un corps d'électrode opposé (8) avec une surface d'électrode opposée (8') librement accessible, sachant que les sections de paroi (7, 7') électriquement isolantes respectivement d'une des surfaces (5, 5') électriquement isolantes de la section d'assemblage (3) de la partie mâle (1) à l'état d'une introduction complète de la section d'assemblage (3) côté partie mâle dans l'ouverture d'introduction (4) sont orientées tournées de telle manière que les surfaces d'électrode opposées (8') touchent respectivement les surfaces d'électrode (6').

14. Connecteur selon l'une quelconque des revendications 1 à 13,
**caractérisé en ce qu'**une normale de surface est respectivement attribuée à la surface (5, 5') électriquement isolante ainsi qu'à la section de paroi (7, 7') électriquement isolante, qui sont orientées de façon orthogonale ou inclinée par rapport au sens d'introduction, le long duquel la section d'assemblage (3) de la partie mâle (1) peut être guidée dans l'ouverture d'introduction (4) de la partie femelle (2) .

15. Connecteur selon l'une quelconque des revendications 1 à 14,
**caractérisé en ce que** la surface d'électrode (6') librement accessible d'au moins un corps d'électrode (6) est orientée parallèlement ou inclinée par rapport à au moins une surface électriquement isolante (5, 5'), et
**en ce que** la surface d'électrode opposée (8') librement accessible d'au moins un corps d'électrode opposé (8) est orientée parallèlement ou inclinée par rapport à au moins une section de paroi (7, 7') électriquement isolante.
